# EUROPÄISCHE PATENTSCHRIFT

(11) **EP 1 495 037 B1**
(45) Veröffentlichungstag und Bekanntmachung des Hinweises auf die Patenterteilung: **25.02.2009**
(21) Anmeldenummer: 03712029.2
(22) Anmeldetag: 17.03.2003
(51) Int. Cl.: C07F 9/50

(54) **VERFAHREN ZUR HERSTELLUNG VON PERFLUORALKYLPHOSPHINEN UND DEREN VERWENDUNG ALS PERFLUORALKYLIERUNGSREAGENZIEN**
METHOD FOR THE PRODUCTION OF PERFLUOROALKYL PHOSPHINES AND THE USE THEREOF AS PERFLUOROALKYLATING REACTANTS
PROCEDE DE PRODUCTION DE PERFLUORO-ALKYLPHOSPHINES ET LEUR UTILISATION COMME REACTIFS DE PERFLUORALKYLATION

(30) Priorität: 18.04.2002 DE 10216998
(43) Veröffentlichungstag der Anmeldung: 12.01.2005
(73) Patentinhaber: Merck Patent GmbH, 64293 Darmstadt (DE)
(72) Erfinder: WELZ-BIERMANN, Urs, 64646 Heppenheim (DE); IGNATYEV, Nikolai, 47058 Duisburg (DE); WEIDEN, Michael, 64285 Darmstadt (DE); SCHMIDT, Michael, 64342 Seeheim-Jugenheim (DE); HEIDER, Udo, Winchester S022 4HZ (GB); MILLER, Alexej, 46058 Duisburg (DE); WILLNER, Helge, 45481 Mühlheim/Ruhr (DE); SARTORI, Peter, 86919 Utting (DE)
(86) Internationale Anmeldenummer: PCT/EP2003/002739
(87) Internationale Veröffentlichungsnummer: WO 2003/087113

(56) Entgegenhaltungen:
- FR-A- 2 827 285
- US-A- 4 701 569
- US-A- 6 096 926
- KAMPA J J: "The synthesis of tris(perfluoroalkyl) phosphanes" ANGEWANDTE CHEMIE. INTERNATIONAL EDITION, VERLAG CHEMIE. WEINHEIM, DE, Bd. 34, Nr. 11, 1995, Seiten 1241-1244, XP002125737 ISSN: 0570-0833 in der Anmeldung erwähnt

## Beschreibung

Die vorliegende Erfindung betrifft ein Verfahren zur Herstellung von Perfluoralkylphosphinen umfassend zumindest die Umsetzung wenigstens eines Fluor(perfluoralkyl)phosphorans mit wenigstens einem Hydrid-Ionen-Donator.

Perfluoralkylphosphine sind an sich bekannt. Nach dem in der Literatur beschriebenen Verfahren werden einige Verbindungen durch Reduktion entsprechender Difluortris(perfluoralkyl)phosphorane mit P[Si(CH₃)₃]₃ gewonnen (J.J. Kampa et al., Angew. Chem., 107, Nr. 11 (1995), Seiten 1334-1337). Nachteilig bei diesem Verfahren ist insbesondere die sehr geringe Ausbeute der Perfluoralkylphosphine sowie die relativ aufwendige Herstellung des Reduktionsmittels P[Si(CH₃)₃]₃.

Die Aufgabe der vorliegenden Erfindung bestand daher darin, ein Verfahren zur Verfügung zu stellen, welches die einfache und kostengünstige Herstellung von Perfluoralkylphosphinen in guten Ausbeuten ermöglicht. Vorzugsweise sollen die Perfluoralkylphosphine in hoher Reinheit erhalten werden.

Diese Aufgabe wurde durch das erfindungsgemäße Verfahren zur Herstellung von Perfluoralkylphosphinen gelöst, welches zumindest die Umsetzung wenigstens eines Fluor(perfluoralkyl)phosphorans mit wenigstens einem Hydrid-Ionen-Donator umfaßt.

Die Herstellung der Fluor(perfluoralkyl)phosphorane kann nach üblichen, dem Fachmann bekannten Methoden erfolgen.
Vorzugsweise werden diese Verbindungen durch elektrochemische Fluorierung geeigneter Ausgangsverbindungen hergestellt, wie in V.Ya. Semenii et al., Zh. Obshch.Khim., 55, Nr. 12 (1985), Seiten 2716-2720; N. Igantiev, P. Sartori, J. of Fluorine Chem., 103 (2000), Seiten 57-61 sowie der WO 00/21969 beschrieben.

Erfindungsgemäß können ein oder mehrere Hyrid-Ionen-Donatoren, d.h. Verbindungen, die in Lage sind, ein oder mehrere Hyrid-Ionen (H⁻) abzugeben, jeweils einzeln oder in Kombination zum Einsatz kommen, wobei der Einsatz jeweils nur eines Hydrid-Ionen-Donators bevorzugt ist.

Vorzugsweise ist der Hydrid-Ionen-Donator ausgewählt aus der Gruppe bestehend aus Hydrosilanen, Alkylhydrosilanen, Metallhydriden, Borhydriden und Hydroboraten.

Sofern als Hydrid-Ionen-Donator ein Alkylhydrosilan in dem erfindungsgemäßen Verfahren zum Einsatz kommt, ist dies bevorzugt Triethylsilan oder Tripropylsilan.

Kommt als Hydrid-Ionen-Donator ein Borhydrid in dem erfindungsgemäßen Verfahren zum Einsatz, ist dies bevorzugt Natriumborhydrid.

In dem erfindungsgemäßen Verfahren wird wenigstens eine Fluor(perfluoralkyl)phosphoranverbindung der allgemeinen Formel I

(CₙF₂ₙ₊₁)ₘPF₅₋ₘ I

mit 1 ≤ n ≤ 8, vorzugsweise 1 ≤ n ≤ 4 und m jeweils gleich 1, 2 oder 3 gemäß dem erfindungsgemäßen Verfahren umgesetzt.

Besonders bevorzugte Fluor(perfluoralkyl)phosphoranverbindungen können ausgewählt werden aus der Gruppe bestehend aus Difluortris(pentafluorethyl)phosphoran, Difluortris(n-nonafluorbutyl)phosphoran und Difluortris(n-heptafluorpropyl)phosphoran, Trifluorbis(n-nonafluorbutyl)phosphoran.

Die Umsetzung wenigstens einer Fluor(perfluoralkyl)phosphoranverbindung kann sowohl in einem geeigneten Reaktionsmedium als auch ohne Gegenwart eines Reaktionsmediums durchgeführt werden. Bevorzugt erfolgt die Umsetzung nach dem erfindungsgemäßen Verfahren ohne Reaktionsmedium, da hierdurch die Umweltbilanz des Verfahrens verbessert und die Kosten reduziert werden.

Die nach dem erfindungsgemäßen Verfahren zum Einsatz kommenden Hydrid-Ionen-Donatoren werden vorzugsweise im Überschuß, jeweils bezogen auf die eingesetzte Menge an Fluor(perfluoralkyl)phosphoran, eingesetzt, um eine vollständige Umsetzung zu den gewünschten Perfluoralkylphosphinen zu gewährleisten. Ebenfalls bevorzugt können die Hydrid-Ionen-Donatoren äquimolar, jeweils bezogen auf die eingesetzte Menge an Fluor(perfluoralkyl)phosphoran, eingesetzt werden.

Die Temperatur während der Umsetzung sowie deren Dauer können bei dem erfindungsgemäßen Verfahren beispielsweise in Abhängigkeit voneinander sowie in Abhängigkeit von dem eingesetzten Fluor(perfluoralkyl)phosphoran und den jeweils gewählten Ansatzgrößen über einen weiten Bereich variieren. Die jeweils optimale Wahl der Parameter kann vom Fachmann durch einfache Vorversuche ermittelt werden.

In einer bevorzugten Ausführungsform des erfindungsgemäßen Verfahrens wird während der Umsetzung des Fluor(perfluoralkyl)phosphorans unter Rückfluß erhitzt.

Die Dauer der Umsetzung beträgt vorzugsweise 0,5 bis 20 Stunden. Sofern Triethylsilan als Hydrid-Ionen-Donator verwendet wird, beträgt die Dauer besonders bevorzugt 3 bis 15 Stunden, für Natriumborhydrid besonders bevorzugt 1 bis 3 Stunden.

An die Herstellung eines oder mehrerer Perfluoralkylphosphine nach dem erfindungsgemäßen Verfahren kann sich, falls erforderlich, eine Reinigung dieser Verbindungen nach üblichen, dem Fachmann bekannten Methoden anschließen.

In einer bevorzugten Ausführungsform des erfindungsgemäßen Verfahrens können die hergestellten Perfluoralkylphosphine durch ein-oder mehrfache Destillation, gegebenenfalls unter vermindertem Druck und/oder ggf. unter Inertgasatmosphäre gereinigt und ggf. isoliert werden.

Das erfindungsgemäße Verfahren zur Herstellung von Perfluoralkylphosphinen ermöglicht die kostengünstige Herstellung dieser Verbindungen in großer Ausbeute und in hohen Reinheiten. Es zeichnet sich ferner dadurch aus, daß die zum Einsatz kommenden Hydrid-Ionen-Donatoren käuflich erhältliche, kostengünstige Verbindungen darstellen, die auch im großtechnischen Maßstab problemlos gehandhabt werden können. Die als Ausgangsverbindungen eingesetzten Fluor(perfluoralkyl)phosphorane können durch elektrochemische Fluorierung ebenfalls kostengünstig hergestellt werden.

Vorteilhaft ist weiterhin, daß das erfindungsgemäße Verfahren lösungsmittelfrei durchgeführt werden kann, was die Herstellungskosten der Perfluoralkylphosphine weiter senkt und die Umweltbilanz des erfindungsgemäßen Verfahrens verbessert.

Die Tris(perfluoralkyl)phosphine eignen sich zur Perfluoralkylierung chemischer Substrate.

Die Perfluoralkylierung stellt ein wichtiges Verfahren zur Herstellung von Fluor-haltigen Verbindungen, insbesondere Organofluorverbindungen dar. Als Perfluoralkylierungsreagenzien werden üblicherweise Perfluoralkylhalogenide, insbesondere Perfluoralkyliodide eingesetzt, die als Quelle von Perfluoralkylradlkalen fungieren ("Organofluorine Chemistry. Principles and Commercial Applications." Edited by R.E. Banks, Plenum Press, New York 1994; G.G. Furin, "Some new aspects in the application of perfluoralkyl halides in the synthesis of fluorine-containing organic compounds" (Review), Russ.Chem.Rev. (English Translation), 69, Nr. 6 (2000), Seiten 491-522; N.O. Brace, "Syntheses with perfluoralkyl iodides. A review, Part III.", J. of Fluorine Chem., 108 (2001), Seiten 147-175; N.O. Brace, "Syntheses with perfluoralkyliodides. Part II.", J. of Fluorine Chem. 96 (1999), Seiten 101-127; N.O. Brace, "Syntheses with perfluoralkyl radicals from perfluoralkyl iodides. A rapid survey of synthetic possibilities with emphasis on practical applications. Part one: alkenes, alkynes and allylic compounds", J. of Fluorine Chem., 96 (1999), Seiten 1-25; V.N. Boiko, "Ion-radical perfluoralkylation. Part II.", J. of Fluorine Chem., 69 (1994), Seiten 207-212).

Darüber hinaus werden Perfluoralkylhalogenide zur Herstellung von Perfluoralkyl-, insbesondere Trifluormethyl-Gruppen enthaltenden Organometallverbindungen eingesetzt, die ihrerseits zur Einführung von Perfluoralkylgruppen in organische Moleküle eingesetzt werden können (D.J. Burton, "Fluorinated organometallics: perfluoralkyl and functionalised perfluoralkyl organometallic reagents in organic synthesis", Tetrahedron, 48, Nr. 2 (1992), Seiten 189-275).

Des weiteren wurde das Reagenz TMSCF₃ zur nucleophilen Trifluormethylierung entwickelt (G.K. Surya Prakash, "Nucleophilic trifluormethylation tamed", J. of Fluorine Chem., 112 (2001), Seiten 123-131). Durch die Reaktion von langkettigen Perfluoralkyliodiden mit Tetrakis(dimethylamino)ethylen in Gegenwart von Chlortrimethylsilan wurde dieses Verfahren der nucleophilen Perfluoralkylierung auf weitere organische und anorganische Substrate ausgedehnt (V.A. Petrov, Tetrahedron Letters, 42 (2001), Seiten 3267-3269).

Die vorstehend angegebenen Verfahren zu Perfluoralkylierung haben jedoch den Nachteil, daß die entsprechenden Perfluoralkylhalogenide entweder sehr teuer sind, oder deren Nutzung, wie beispielsweise im Falle der Verbindung CF₃Br, nach dem Montreal Protokoll nur sehr eingeschränkt gestattet ist.

Diese Nachteile führten zu der Entwicklung neuer Perfluoralkylierungsreagenzien, wie sie in J.R. Desmurs et al., 12th European Symposium on Fluorine Chemistry, Berlin, Germany, 1998, Abstracts A23 and A24 beschrieben sind. Die Herstellung dieser Reagenzien gelingt allerdings nur unter Verwendung von CF₃H, einer hochflüchtigen und schwer handhabbaren Verbindung. Ferner wurden weitere stabile Perfluoralkylierungsreagenzlen zur nucleophilen Triluormethylierung entwickelt, wobei zur Synthese dieser Reagenzien vom Methylhemiketal des Fluorals ausgegangen wird, welches zuvor in einem relativ aufwendigen Prozeß hergestellt werden muß. Außerdem ist die Anwendung dieser Reagenzien auf die Trifluormethylierung beschränkt (G.Blond et al., Tetrahedron Letters, 42 (2001), Seiten 2437-2475; T. Billard et al., Eur. J. Org. Chem., 2001, Seiten 1467-1471; T. Billard et al. Tetrahedron Letters, 41 (2000), Seiten 8777-8780; G. Blond et al., J. Org. Chem., 66, Nr. 14 (2001), Seiten 4826-4830).

Zur Perfluoralkylierung von chemischen Substraten mit Perfluoralkylphosphinen ist es erforderlich, das zu perfluoralkylierende Substrat entweder vor oder während der Umsetzung mit dem jeweiligen Perfluoralkylphosphin mit wenigstens einer Base zu behandeln. Bevorzugt erfolgt die Perfluoralkylierung des chemischen Substrates mit wenigstens einem Perfluoralkylphosphin in Gegenwart wenigstens einer Base.

Bevorzugt kommen hierfür starke Basen, wie beispielsweise Kalium tert-Butylat, n-Butyllithium und/oder ein Grignard-Reagenz in Betracht.

Vorzugsweise wird die Perfluoralkylierung in einem geeigneten, ggf. nach üblichen Verfahren getrocknetem Reaktionsmedium, wie beispielsweise cyclischen oder aliphatischen Ether, insbesondere Tetrahydrofuran oder Diethylether, durchgeführt.

Als chemische Substrate kommen bevorzugt organische Verbindungen, insbesondere dreifach koordinierte Organborverbindungen sowie Carbonyl-Gruppen aufweisende organische Verbindungen in Betracht.

Als Organoborverbindungen kommen bevorzugt Tris-(C₁₋₃)-Alkylborate, besonders bevorzugt Trimethylborat zum Einsatz.

Bevorzugte Carbonylgruppen-aufweisende Verbindungen sind ggf. substituierte Diarylketonverbindungen, insbesondere Benzophenon.

Vorzugsweise kann die Perfluralkylierung chemischer Substrate mit Perfluoralkylphosphinen unter Inertgasatmosphäre, wie z.B. Argon oder Stickstoff, durchgeführt werden.

Die Verwendung von Tris(perfluoralkyl)phosphinen als Perfluoralkylierungsreagenzien ist insbesondere deshalb vorteilhaft, weil diese Verbindungen, im Gegensatz zu vielen anderen Perfluoralkylierungsreagenzien, stabile Verbindungen darstellen, was ihre einfache, sichere Handhabung ermöglicht.

Die NMR-Spektren wurden mit Hilfe eines Bruker Avance 300 NMR Spektrometers mit folgenden Frequenzen aufgenommen:
300,1 MHz für ¹H
282,4 MHz für ¹⁹F und
96,3 MHz für ¹¹B.

Die Massenspektren wurden mit einem Gerät vom Typ AMD 604 gemessen.

Im folgenden wird die Erfindung anhand von Beispielen erläutert. Diese Beispiele dienen lediglich der Erläuterung der Erfindung und schränken den allgemeinen Erfindungsgedanken nicht ein.

### Beispiele

### Beispiel 1a:

### Herstellung von Tris(pentafluorethyl)phosphin

In einem FEP(Fluorethylenpolymer)-Kolben wurden 56,0 g (131,4 mmol) Difluortris(pentafluorethyl)phosphoran und 38,0 g (326,8 mmol) Triethylsilan unter heftigem Rühren für 12 Stunden bei einer Badtemperatur von 110 °C am Rückfluß erhitzt. Anschließend wurde das Reaktionsgemisch unter Normaldruck unter einer Inertgasatmosphäre destilliert und 48,0 g der Fraktion mit einem Siedebereich von 81-85 °C aufgefangen. Diese Fraktion wurde anschließend auf -20°C abgekühlt und die untere Phase (gewünschtes Produkt) abgetrennt. Es wurden 42,2 g von nahezu reinem Tris(pentafluorethyl)phosphin erhalten, entsprechend einer Ausbeute von 82,8 %, bezogen auf das eingesetzte Difluortris(pentafluorethyl)phosphoran.

Das so erhaltene Produkt wurde mittels ¹⁹F-, ³¹P- und ¹H-NMR-Spektroskopie sowie durch Massenspektroskopie charakterisiert:
¹⁹F NMR Spektrum; δ, ppm:
   (Lösungsmittel CDCl₃, interne Referenz CCl₃F)
   -82,4 dt (CF₃); -106,5 dq (CF₂); J²_{P,F} = 49,3 Hz; J³_{P,F} = 15,8 Hz; J³_{F,F} = 3,1 Hz
³¹P NMR Spektrum; δ, ppm:
   (Lösungsmittel CDCl₃, Referenz 85 Gew.-%ige H₃PO₄)
   13,3 sep of dec

   Die Werte der gefundenen chemischen Verschiebungen entsprechen den aus der Veröffentlichung von J.J. Kampa et al., Angew. Chem., 107, Nr. 11 (1995), Seiten 1334-1337 bekannten Werten.
Hochauflösendes Massenspektrum:
   Berechnet (M+): 387,949812
   Gefunden (M+): 387,949842

### Beispiel 1b:

### Herstellung von Tris(pentafluorethyl)phosphin

In einem Glaskolben wurden 230,0 g (0,54 mol) Difluortris(pentafluorethyl)phosphoran und 41,2 g (1,089 mol) Natriumborhydrid unter heftigem Rühren für 3 Stunden bei einer Badtemperatur von ca. 110 °C unter Rückfluß erhitzt. Anschließend wurde das Reaktionsgemisch unter vermindertem Druck (2 KPa) destilliert und zwei Fraktionen des Produkts in Kühlfallen mit einer Temperatur von -78°C bzw. - 195 °C aufgefangen. Zur weiteren Reinigung wurden die vereinigten Produkte von beiden Fällen bei Normaldruck unter Inertgas-Atmosphäre destilliert, wobei die Fraktion im Siedebereich 85-87 °C aufgefangen wurde. Es wurden 194,0 g reines Tris(pentafluorethyl)phosphin erhalten. Die Ausbeute betrug 93 %, bezogen auf die Menge an eingesetztem Difluortris(pentafluorethyl)-phosphoran.
Das so erhaltene Produkt wurde mittels ¹⁹F- und ³¹P-NMR-Spektroskopie charakterisiert. Die gefundenen chemischen Verschiebungen entsprechen denen aus Beispiel 1a.

### Beispiel 2:

### Herstellung von Tris(n-nonafluorbutyl)phosphin

In einem FEP-Kolben wurden 19,7 g (27,13 mmol) Difluortris(n-nonafluor butyl)phosphoran und 10,0 g (86,0 mmol) Triethylsilan unter heftigem Rühren für 15 Stunden bei einer Badtemperatur von ca. 140 °C unter Rückfluß erhitzt. Anschließend wurde das Reaktionsgemisch unter vermindertem Druck (1,73 kPa) destilliert und die Fraktion mit einem Siedepunkt von 87 °C aufgefangen. Es wurden 15,0 g der klaren, farblosen Flüssigkeit von Tris(n-nonafluorbutyl)phosphin erhalten. Die Ausbeute betrug 80,3 %, bezogen auf die eingesetzte Menge an Difluortris(n-nonafluorbutyl)phosphoran.

Das so erhaltene Produkt wurde mittels ¹⁹F- und ³¹P-NMR-Spektroskopie sowie durch Elementaranalyse charakterisiert:
¹⁹F NMR Spektrum; δ, ppm:
   (Lösungsmittel CDCl₃, interne Referenz CCl₃F)
   -81,4 t (CF₃); -102,2 dm (CF₂); -118,9 dm (CF₂); -126,3 m (CF₂); J²_{P,F} = 37,7 Hz; J³_{P,F} = 35,2 Hz; J⁴_{F,F} = 9,6 Hz
³¹P NMR Spektrum; δ, ppm:
   (Lösungsmittel CDCl₃, Referenz 85 Gew.-%ige H₃PO₄) 23,3 m

Die Werte der gefundenen chemischen Verschiebungen entsprechen den aus der Veröffentlichung von J.J. Kampa et al., Angew. Chem., 107, Nr. 11 (1995), Seiten 1334-1337 bekannten Werten.

### Beispiel 3:

### Herstellung von Bis(n-nonafluorbutyl)phosphin

In einem FEP-Kolben wurden 4,6 g (8,7 mmol) Trifluorbis(n-nonafluorbutyl)phosphoran und 4,32 g (27,3 mmol) Tripropylsilan unter heftigem Rühren für 3 Stunden bei einer Badtemperatur von ca. 70 °C unter Rückfluß erhitzt. Anschließend wurde das Reaktionsgemisch bei Normaldruck und unter Inertgas-Atmosphäre destilliert und die Fraktion im Siedebereich von 130-135 °C aufgefangen. Es wurden 3,0 g der klaren, farblosen Flüssigkeit von Bis(n-nonafluorbutyl)phosphin erhalten. Die Ausbeute betrug 73,3 %, bezogen auf die eingesetzte Menge an Trifluorbis(n-nonafluorbutyl)phosphoran.

Das so erhaltene Produkt wurde mittels ¹⁹F- und ³¹P-NMR-Spektroskopie charakterisiert. Die Spektren wurden für die reine Flüssigkeit unter Verwendung einer FEP-Röhre mit einem Acetonitril-D₃ Film als externer "lock" und CCl₃F oder 85 % H₃PO₄ in D₂O als externe Referenz gemessen.
¹⁹F NMR Spektrum; δ, ppm:
   - 81,6 m (CF₃); -120,2 m (CF₂); -121,1 m (CF₂); -126,0 m (CF₂)
³¹P NMR Spektrum; δ, ppm:
   140,0 dm; J¹_{P,H} = 1025 Hz

### Beispiel 4:

### Perfluoralkylierung von Benzophenon

5,81 g (14,97 mmol) Tris(pentafluorethyl)phosphin wurden unter Inertgas-Atmosphäre zu einer Lösung von 1,68 g (14,97 mmol) Kalium-tert.-Butylat und 2,72 (14,93 mmol) Benzophenon in 20 cm³ getrocknetem Tetrahydrofuran unter Kühlung mit einem Wasserbad so zugegeben, daß die Temperatur des Reaktionsgemisches bei ca. 20 °C lag. Anschließend wurde die Reaktionsmischung eine Stunde bei Raumtemperatur nachgerührt, mit 20 cm³ einer 0,1 N Lösung von HCl versetzt und zweimal mit jeweils 75 cm³ Dietehylether extrahiert. Die vereinigten Extrakte wurden dreimal mit jeweils 20 cm³ Wasser gewaschen und über wasserfreiem Magnesiumsulfat getrocknet Das Lösungsmittel wurde anschließend abdestilliert und das gewünschte Produkt aus n-Hexan auskristallisiert. Es wurden 2,8 g
2,2,3,3,3-Pentafluor-1,1-diphenylpropan-1-ol mit einem Schmelzpunkt im Bereich von 82-83 °C erhalten.
Die Ausbeute betrug 62 %, bezogen auf die eingesetzte Menge an Benzophenon.

Das so erhaltene Produkt wurde mittels ¹⁹F- und ¹H-NMR-Spektroskopie sowie durch hoch auflösende Massenspektroskopie und Elementaranalyse charakterisiert:
¹⁹F NMR Spektrum; δ, ppm:
   (Lösungsmittel CDCl₃, interne Referenz CCl₃F)
   -77,6 s (CF₃); -116,9 m (CF₂)
¹H-NMR Spektrum; δ, ppm:
   (Lösungsmittel CDCl₃, Referenz TMS)
   7,53-7,67 m (2H), 7,30-7,47 m (3H), 2,85 br.s (OH)

   Der Schmelzpunkt sowie die gefundenen chemischen Verschiebungen stimmen mit den aus der Veröffentlichung von L.S. Chen et al.; J. of Fluorine Chem., 20 (1982), Seiten 341-348 bekannten Werten überein.
Hochauflösendes Massenspektrum:
   Berechnet (M+): 302,073006
   Gefunden (M+): 302,073115
Elementaranalyse:
   Gefunden; C 59,67 %; H 3,62 %
   Berechnet für (C₆H₅)₂C(OH)C₂F₅: C 59,61 %; H 3,67 %

### Beispiel 5:

### Kaliumpentafluorethyltrifluorborat (C₂F₅)BF₃K

a) 9,78 g (25,21 mmol) Tris(pentafluorethyl)phosphin wurden langsam unter Inertgas-Atmosphäre zu einer gerührten Lösung von 30 mmol n-Butyllithium (15 cm³ einer 2 M Lösung in Cyclohexan) in 70 cm³ getrocknetem Diethylether unter Kühlung so zugegeben, daß die Temperatur des Reaktionsgemisches bei ca. - 60 °C lag. Anschließend wurde die Reaktionsmischung 30 Minuten bei - 55 °C nachgerührt und eine Lösung von 3,31 g (31,85 mmol) Trimethylborat (CH₃O)₃B in 5 cm³ getrocknetem Diethylether zugegeben. Die so erhaltene Reaktionsmischung wurde auf Raumtemperatur gebracht und mit 30 cm³ Flusssäure (48 %ige wäßrige Lösung) versetzt und anschließend weitere 10 Stunden bei Raumtemperatur gerührt. Im Anschluß daran wurden 10,0 g (128 mmol) Kaliumhydrogendifluorid zugegeben und weitere 10 Stunden bei Raumtemperatur gerührt. Die organische Phase wurde abgetrennt, durch Rühren über festem Kaliumcarbonat neutralisiert und mit wasserfreiem Magnesiumsulfat getrocknet. Das Lösungsmittel wurde anschließend abdestilliert und der so erhaltene Rückstand in einer möglichst geringen Menge Tetrahydrofuran gelöst. Durch Zugabe von Chloroform wurde dann ein Feststoff ausgefällt, der abfiltriert und getrocknet wurde. Es wurden 3,30 g Kaliumpentafluorethyltrifluorborat in Form eines weißen Feststoffes erhalten. Die Ausbeute betrug 58 %, bezogen auf das eingesetzte Tris(pentafluorethyl)phosphin.
   Das so erhaltene Produkt wurde mittels ¹¹B- und ¹⁹F-NMR-Spektroskopie sowie durch Elementaranalyse charakterisiert:
   ¹¹B-NMR Spektrum; δ, ppm:
      (Lösungsmittel Aceton-D6, externe Referenz BF₃ O(C₂H₅)₂):
      0,2 tq; J¹_{B,F} = 41,0 Hz; J²_{B,F} = 20,0 Hz
   ¹⁹F NMR Spektrum; δ, ppm:
      (Lösungsmittel Aceton-D6, interne Referenz CCl₃F)
      -83,3 q (CF₃); -136,2 q, (CF₂); -153,5 q (BF₃); J¹_{B,F} = 41,2 Hz; J²_{B,F} = 19,9 Hz; J⁴_{F,F} = 4,9 Hz.
   Elementaranalyse:
      Gefunden: C 10,56 %
      Berechnet (für (C₂F₅)BF₃K): C 10,63 %
b) Kaliumpentafluorethyltrifluorborat (C₂F₅)BF₃K

20,81 g (53,63 mmol) Tris(pentafluorethyl)phosphin wurden unter Inertgasatmosphäre langsam zu einer gerührten Lösung von 60 mmol Methylmagnesiumchlorid (20 cm³ einer 3 M Lösung in Tetrahydrofuran) in 100 cm³ getrocknetem Tetrahydrofuran unter Kühlung so zugegeben, daß die Temperatur des Reaktionsgemisches bei ca. -60 °C lag. Anschließend wurde die Reaktionsmischung 30 Minuten bei - 55 °C nachgerührt und 6,24 g (60,04 mmol) Trimethylborat (CH₃O)₃B zugegeben. Die so erhaltene Reaktionsmischung wurde auf Raumtemperatur gebracht und mit 25 cm³ Flusssäure (48 %ige wäßrige Lösung) versetzt und anschließend weitere 10 Stunden bei Raumtemperatur gerührt.
Im Anschluß daran wurden 15,0 g (192 mmol) Kaliumhydrogendifluorid zugegeben und weitere 10 Stunden bei Raumtemperatur gerührt. Die organische Phase wurde abgetrennt, durch Rühren über festem Kaliumcarbonat neutralisiert und mit wasserfreiem Magnesiumsulfat getrocknet. Das Lösungsmittel wurde anschließend abdestilliert und der so erhaltene Rückstand in einer möglichst geringen Menge Tetrahydrofuran gelöst. Durch Zugabe von Chloroform wurde dann ein Feststoff ausgefällt, der abfiltriert und getrocknet wurde. Es wurden 6,30 g Kaliumpentafluorethyltrifluorborat in Form eines weißen Feststoffes erhalten. Die Ausbeute betrug 52 %, bezogen auf das eingesetzte Tris(pentafluorethyl)phosphin.

Das so erhaltene Produkt wurde mittels ¹¹B- und ¹⁹F-NMR-Spektroskopie charakterisiert. Die entsprechenden Signale stimmten mit den unter 5a genannten Signalen überein.

### Beispiel 6:

1,925 g (20,53 mmol) Lithiumtetrafluorborat, LiBF₄ wurden in 10 cm³ trockenem Dimethylcarbonat gelöst. Unter Rühren wurde bei Raumtemperatur eine Lösung aus 4,639 g (20,53 mmol) Kaliumpentafluorethyltrifluorborat (C₂F₅)BF₃K in 19 cm₃ trockenem Dimethylcarbonat hinzugegeben. Der weiße Niederschlag von KBF₄ wurde abfiltriert. Das Lösungsmittel wurde bei verringertem Druck (1,3 Pa) entfernt. Der Rückstand (5,525 g, Ausbeute: 95 %) bestand aus Lithiumpentafluorethyltrifluorborat als Komplex mit Dimethylcarbonat (1:1) (C₂F₅)BF₃Li · (CH₃O)₂CO.
Die Struktur wurde durch die ¹⁹F-, ¹¹B- und ¹H-NMR-Spektren bestätigt.
¹H-NMR-Spektrum, δ, ppm
(Lösungsmittel: Acetonitril-d₃, Referenz: TMS):
3,74 s, (CH₃O)₂CO.
¹¹B-NMR-Spektrum, δ, ppm
(Lösungsmittel: Acetonitril-d₃, Referenz: BF₃ . OEt₂-extern)
-0,1 tq; J¹_{B,F}= 40,4 Hz; J²_{B,F}=19,9 Hz.
¹⁹F-NMR-Spektrum, δ, ppm
(Lösungsmittel: Acetonitril-d₃, Referenz: CCl3F -intern)
-83,27 q (CF₃); -136,14 q (CF₂), -154,03 q (BF₃); J¹_{B,F}=41,2 Hz; J²_{B,F}=19,9 Hz; J⁴_{F,F}=4,9 Hz.

## Patentansprüche

1. Verfahren zur Herstellung von Perfluoralkylphosphinen, **dadurch gekennzeichnet, daß** es zumindest die Umsetzung wenigstens eines Fluor(perfluoralkyl)phosphorans der allgemeinen Formel I
(CₙF₂ₙ₊₁)ₘPF₅₋ₘ (I)
worin 1 ≤ n ≤ 8, vorzugsweise 1 ≤ n ≤4 und m jeweils gleich 1, 2 oder 3 bedeutet, mit wenigstens einem Hydrid-Ionen-Donator umfaßt.

2. Verfahren gemäß Anspruch 1, **dadurch gekennzeichnet, daß** als Fluor(perfluoralkyl)phosphoran eine Verbindung ausgewählt aus der Gruppe bestehend aus Difluortris(pentafluorethyl)phosphoran, Difluortris(n-nonafluorbutyl)phosphoran, Trifluorbis(n-nonafluorbutyl)phosphoran und Difluortris(n-heptafluorpropyl)phosphoran eingesetzt wird.

3. Verfahren gemäß Anspruch 1 oder 2, **dadurch gekennzeichnet, daß** die Reduktion ohne Reaktionsmedium durchgeführt wird.

4. Verfahren gemäß einem der Ansprüche 1 bis 3, **dadurch gekennzeichnet, daß** der Hydrid-Ionen-Donator eine Verbindung ausgewählt aus der Gruppe bestehend aus Hydrosilanen, Alkylhydrosilanen, Metallhydriden, Borhydriden und Hydroboraten ist. -

5. Verfahren gemäß Anspruch 4, **dadurch gekennzeichnet, daß** das Alkylhydrosilan Triethylsilan oder Tripropylsilan ist.

6. Verfahren gemäß Anspruch 4, **dadurch gekennzeichnet, daß** das Borhydrid Natriumborhydrid ist.

7. Verfahren gemäß einem der Ansprüche 1 bis 6, **dadurch gekennzeichnet, daß** der Hydrid-Ionen-Donator äquimolar oder im Überschuß, jeweils bezogen auf die eingesetzte Menge an Fluor(perfluoralkyl)phosphoran, eingesetzt wird.

8. Verfahren gemäß einem der Ansprüche 1 bis 7, **dadurch gekennzeichnet, daß** während der Umsetzung unter Rückfluß erhitzt wird.

9. Verfahren gemäß einem der Ansprüche 1 bis 8, **dadurch gekennzeichnet, daß** die Dauer der Umsetzung 0,5 bis 20 Stunden, vorzugsweise 1 bis 15 Stunden beträgt.

10. Verfahren gemäß einem der Ansprüche 1 bis 9, **dadurch gekennzeichnet, daß** das/die Perfluoralkylphosphin(e) durch Destillation, vorzugsweise unter Inertgasatmosphäre, gegebenenfalls unter vermindertem Druck gereinigt wird/werden.

## Claims

1. Process for the preparation of perfluoroalkylphosphines, **characterised in that** it comprises at least the reaction of at least one fluoro(perfluoroalkyl)phosphorane of the general formula I
(CₙF₂ₙ₊₁)ₘPF₅₋ₘ, (I)
in which 1 ≤ n ≤ 8, preferably 1 ≤ n ≤ 4, and m in each case denotes 1, 2 or 3, with at least one hydride ion donor.

2. Process according to Claim 1, **characterised in that** the fluoro-(perfluoroalkyl)phosphorane employed is a compound selected from the group consisting of difluorotris(pentafluoroethyl)phosphorane, difluorotris(n-nonafluorobutyl)phosphorane, trifluoro-bis(n-nonafluorobutyl)phosphorane and difluorotris(n-heptafluoropropyl)phosphorane.

3. Process according to Claim 1 or 2, **characterised in that** the reduction is carried out without reaction medium.

4. Process according to one of Claims 1 to 3, **characterised in that** the hydride ion donor is a compound selected from the group consisting of hydrosilanes, alkylhydrosilanes, metal hydrides, borohydrides and hydroborates.

5. Process according to Claim 4, **characterised in that** the alkylhydrosilane is triethylsilane or tripropylsilane.

6. Process according to Claim 4, **characterised in that** the borohydride is sodium borohydride.

7. Process according to one of Claims 1 to 6, **characterised in that** the hydride ion donor is employed in an equimolar amount or in excess, in each case based on the amount of fluoro(perfluoroalkyl)phosphorane employed.

8. Process according to one of Claims 1 to 7, **characterised in that** the reaction mixture is refluxed during the reaction.

9. Process according to one of Claims 1 to 8, **characterised in that** the duration of the reaction is 0.5 to 20 hours, preferably 1 to 15 hours.

10. Process according to one of Claims 1 to 9, **characterised in that** the perfluoroalkylphosphine(s) is (are) purified by distillation, preferably under an inert-gas atmosphere, if desired under reduced pressure.

## Revendications

1. Procédé de préparation de perfluoroalkylphosphines, **caractérisé en ce qu'**il comprend au moins la réaction d'au moins un fluoro-(perfluoroalkyl)phosphorane de formule générale I
(CₙF₂ₙ₊₁)ₘPF₅₋ₘ, (I)
dans laquelle 1 ≤ n ≤ 8, préférablement 1 ≤ n ≤ 4, et m dans chaque cas désigne 1, 2 ou 3, avec au moins un donneur d'ion hydrure.

2. Procédé selon la revendication 1, **caractérisé en ce que** le fluoro(perfluoroalkyl)phosphorane employé est un composé choisi parmi le groupe constitué par le difluorotris(pentafluoroéthyl)phosphorane, le difluorotris(n-nonafluorobutyl)phosphorane, le trifluorobis(n-nonafluorobutyl)phosphorane et le difluorotris(n-heptafluoropropyl)phosphorane.

3. Procédé selon la revendication 1 ou 2, **caractérisé en ce que** la réduction est effectuée sans milieu de réaction.

4. Procédé selon l'une parmi les revendications 1 à 3, **caractérisé en ce que** le donneur d'ion hydrure est un composé choisi parmi le groupe constitué par les hydrosilanes, les alkylhydrosilanes, les hydrures métalliques, les borohydrures et les hydroborates.

5. Procédé selon la revendication 4, **caractérisé en ce que** l'alkylhydrosilane est le triéthylsilane ou le tripropylsilane.

6. Procédé selon la revendication 4, **caractérisé en ce que** le borohydrure est le borohydrure de sodium.

7. Procédé selon l'une parmi les revendications 1 à 6, **caractérisé en ce que** le donneur d'ion hydrure est employé selon une quantité équimolaire ou en excès, dans chaque cas sur la base de la quantité de fluoro(perfluoroalkyl)phosphorane employée.

8. Procédé selon l'une parmi les revendications 1 à 7, **caractérisé en ce que** le mélange réactionnel est mis au reflux pendant la réaction.

9. Procédé selon l'une parmi les revendications 1 à 8, **caractérisé en ce que** la durée de la réaction va de 0,5 à 20 heures, préférablement de 1 à 15 heures.

10. Procédé selon l'une parmi les revendications 1 à 9, **caractérisé en ce que** le ou les perfluoroalkylphosphine(s) est (sont) purifiées par distillation, préférablement sous une atmosphère de gaz inerte, si on le souhaite sous pression réduite.
